Europäisches Patentamt

European Patent Office

Office européen des brevets

(11) Publication number: **0 256 171**
**A1**

(12) **EUROPEAN PATENT APPLICATION**

(21) Application number: 86201441.2

(22) Date of filing: 20.08.86

(51) Int. Cl.³: **F 17 C 13/02**
**//A61M16/00**

(43) Date of publication of application:
24.02.88 Bulletin 88/8

(84) Designated Contracting States:
AT BE CH DE FR GB IT LI LU NL SE

(71) Applicant: N.V. W.A. Hoek's Machine- en Zuurstoffabriek
Transformatorweg 40
NL-1014 AK Amsterdam(NL)

(72) Inventor: Van Aalst, Nelis
Klipper 54
NL-2991 KL Barendrecht(NL)

(72) Inventor: Braber, Jan
Thorbeckestraat 37
NL-2613 BT Delft(NL)

(72) Inventor: Opentij, Gerardus Arend
Grift 63
NL-1423 DJ Uithoorn(NL)

(72) Inventor: Steenbrugge, Maarten
Tuinhof 28
NL-1441 BN Purmerend(NL)

(74) Representative: de Vries, Johannes Hendrik
Fokke et al,
Octrooibureau Los en Stigter B.V. P.O. Box 20052
NL-1000 HB Amsterdam(NL)

(54) **Apparatus for measuring and delivering predetermined quantities of a compressible medium.**

(57) A device for measuring and delivering of specified quantaties of compressible fluid comprising a fluid container and adjustment means which determine a quantity of fluid to be delivered. The fluid container consist of at least one pressure vessel 2 having a fixed volume, the pressure of the fluid herein being measurable by a pressure transducer 9. The adjustment means are valve members 7, 10 accommodated in a gas line before and after the pressure vessel. During filling of the pressure vessel with fluid the valve member located before the pressure vessel is closed at a predetermined upper pressure and during emptying of the pressure vessel the valve member located after the pressure vessel is closed at a predetermined lower pressure by means of the pressure transducer (9).

fig.1

Croydon Printing Company Ltd

EP 0 256 171 A1

Device for measuring and delivering specified quantities of compressible fluid

The invention relates to a device for measuring and delivering specified quantaties of compressible fluid, comprising a fluid container and adjusting means determining the quantity of fluid to be delivered.

In a known embodiment of such a device, which is used in a so-called lung ventilator with which medical gas is supplied to a patient, the fluid container consists of a bag, which is operated mechanically or pneumatically. The adjusting means constitute of adjustable stops, which determine the inward and outward stroke of the bag and therefore the quantity of fluid to be delivered.

However, this known device has the disadvantage, that the measurement and adjustment of the stroke of the bag is rather inaccurate, which is inadmissible under certain circumstances in practice.

It is an object of the present invention to provide a device of the kind mentioned in the preamble, wherein the disadvantage described hereinbefore is removed in an effective way and wherein a very accurate determination of the quantity of compressible fluid to be delivered is effected.

For this purpose the device according to the invention is characterized in that the fluid container comprises at least one pressure vessel having a fixed volume, the pressure of the fluid therein being measurable by a pressure transducer, the adjustment means being valve members accommodated in a gas line before and after the pressure vessel, wherein during filling of the pressure vessel with fluid the valve member located before the pressure vessel is closed at a predetermined upper pressure and during emptying of the pressure vessel the valve member located after the pressure vessel is closed at a predetermined lower pressure by means of the pressure transducer.

In this way the quantity of fluid to be delivered is determined by the difference between the upper and lower pressures in combination with the fixed volume of the pressure vessel. As it is possible to measure the pressure with great accuracy, while also the volume of the pressure vessel will be known very precisely, a simple yet extremely effective measurement of the quantity of fluid to be delivered can be realised.

According to a favourable embodiment of the device according to the invention a number of pressure vessels having different volumes are arranged in a parallel connection, the pressure vessels each being optionally applicable.

Consequently it is made very easy to choose within a variaty of quantities of fluid to be delivered a suitable pressure vessel, in which the upper and lower pressures differ sufficiently to enable an accurate measurement.

In an advantageous embodiment of the device according to the invention an adjustable throttle member is accommodated in the gas line after the pressure vessel, and preferably the adjustable throttle member is a controllable restriction.

In this way it is enabled to vary the time in which the quantity of fluid is delivered by means of the adjustable throttle member.

Herein it is advantageously if a pressure regulator is accommodated in the gas line between the pressure vessel and the adjustable throttle member.

This pressure regulator serves for keeping the pressure before the throttle member constant when the pressure vessel empties itself. As a result the rate of gas flow through the throttle member will remain constant, so that a uniform delivery of the fluid is effected.

A favourable embodiment of the device according to the invention is characterized in that the pressure vessel contains a material having good heat conducting properties and having a large contacting area.

This heat conducting material serves as heat buffer in the pressure vessel. The heat released through the compression of the fluid when the pressure vessel is filled is absorbed by the heat conducting material and is delivered again to the fluid with the compression relieve when the pressure vessel is drained, so that the temperature thereof remains constant. Herethrough the measured pressure is a very accurate measure for the quantity of the fluid delivered.

According to a favourable embodiment of the device according to the invention the pressure transducer and the valve members are connected to a microcomputer.

In this case a very good and fully automatic control of the device is possible.

The invention will hereafter be elucidated with reference to the drawing, which shows several embodiments of the device according to the invention.

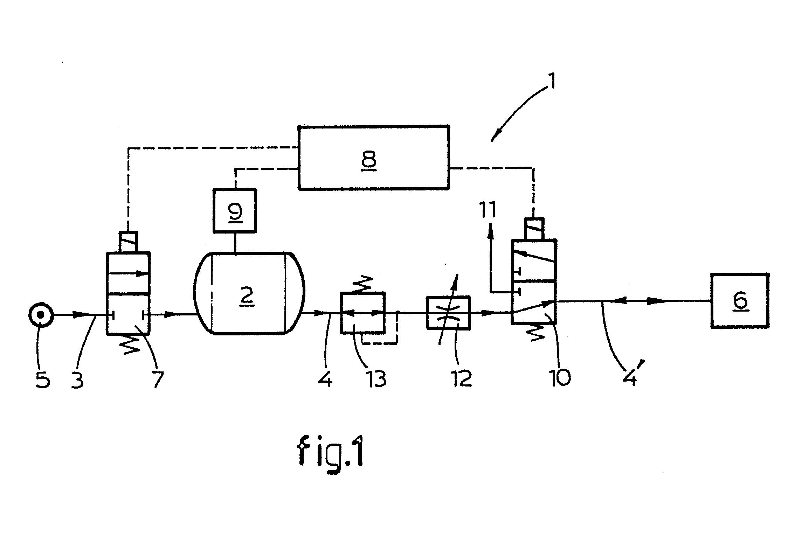

Fig. 1, 2 and 3 show schematic diagrams of three embodiments of the device according to the invention for measuring and delivering specified quantities of compressible fluid.

In the drawing like parts are indicated with like reference numerals.

Fig. 1 illustrates a first embodiment of a device 1 for measuring and delivering specified quantities of compressible fluid.

Such a device 1 is used for instance in so-called 'lung ventilators'. Herein a medical gas is supplied to a patient in a specified quantity and in a specified frequency.

The device 1 comprises a pressure vessel 2, to which a gas supply line 3 and a gas discharge line 4 are connected. The gas supply line 3 is fed with the medical gas by a source 5, the medical gas being supplied to the patient 6 through the device 1.

The medical gas may comprise of a mixture of oxygen and one or more other gases, such as laughing-gas, carbon dioxide, cyclopropane, or of aenastatic vapours. The medical gas, however, may also consist of air for breathing upon the patient.

In the supply line 3 an electromagnetic actuated valve 7 is accommodated, which is controlled by an electronic control unit 8, for instance a microcomputer.

A pressure transducer 9 is also connected to said control unit 8, the pressure transducer being able to measure the pressure of the medical gas in the pressure vessel 2 and to transduce this pressure into an electrical signal to be delivered to the control unit 8.

In the discharge line 4 a valve 10 controlled by the control unit 8 is accommodated, the valve 10 enabling to cut off the gas flow supplied to the patient 6 through a line 4', while the air expired by the patient 6 through the same line 4' may be discharged at an outlet 11 of the valve 10.

In the line 4 between the pressure vessel 2 and the valve 10 are furthermore an adjustable throttle member 12, such as a controllable restriction, and a pressure regulator 13.

The throttle member 12 enables the rate of flow of the gas to be adjusted, so that it is possible to vary the time within which the predetermined quantity of gas is supplied to the patient.

During draining of the pressure vessel 2 the pressure herein decreases, so that also the rate of gas flow decreases. However, it is desirable to maintain a constant rate of gas flow during the whole outflow period. This can be obtained by means of the pressure regulator 13, which keeps the pressure before the throttle member 12 constant, so that also the rate of gas flow remains constant.

The device 1 works as follows:

At the beginning of a cycle both valves 7 and 10 are closed for the medical gas. When the valve 7 is opened the medical gas flows from the source 5 into the pressure vessel 2, wherein the pressure transducer 9 measures the pressure thereof. When a predetermined upper pressure is measured the electronic control unit 8 responds such, that the electromagnetic valve 7 is closed by the control unit 8. Thereupon the valve 10 is opened and the medical gas flows through the lines 4 and 4' to the lungs of the patient 6. When the pressure in the pressure vessel 2 measured by the pressure transducer 9 has reached a predetermined lower pressure the control unit 8 will cause the valve 10 to be closed.

During the expiring of the patient 6, wherein the expired air is discharged through the line 4' out of the outlet 11 of the valve 10, the valve 7 is opened again and the pressure vessel 2 is refilled with new medical gas.

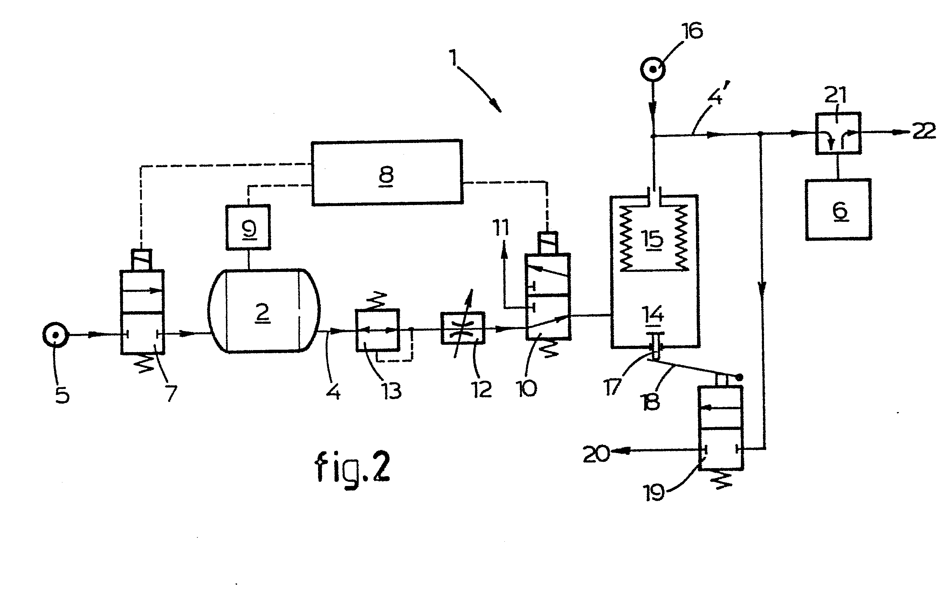

Fig. 2 shows a second embodiment of the device 1, wherein the supply of the medical gas is effected through the so called 'bag-in-bottle' principle. Herein the gas flowing through the device 1 is separated from the medical gas supplied to the patient, so that the gas in the device 1 only serves as propellant and may therefore consist of any gas, such as compressed air or oxygen.

The gas from the pressure vessel 2 is supplied to a pressure chamber 14 (bottle), in which a thin-walled flexible balloon or a folding bag or bellows 15 (bag) is provided, which is adapted to contain the medical gas to be supplied to the patient. The medical gas is supplied from a source 16 to the bag 15.

In the pressure chamber 14 a switch 17 is provided, which is adapted to be activated by the bag 15 thereby actuating a lever 18 of an overflow 19.

The switch 17 is activated when the bag 15 has reached its maximum volume during the inflow of the medical gas, wherein the lower end of the bag 15 comes into engagement with the switch 17. The lever 18 actuated by the switch 17 then opens the overflow valve 19, so that a surplus of medical gas can be discharged at an outlet 20.

When the bag 15 is filled with medical gas it is possible for the propellant in the pressure chamber 14 to drain off at the outlet 11 of the valve 10.

To supply the medical gas to the patient 6 the propellant is released from the pressure vessel 2 into the pressure chamber 14, so that the bag 15 is compressed, and the medical gas flows from the bag 15 through a one way valve 21 to the patient 6. The expired air from the patient 6 is discharged from the system at 22, so that this system is indicated as an 'open system'.

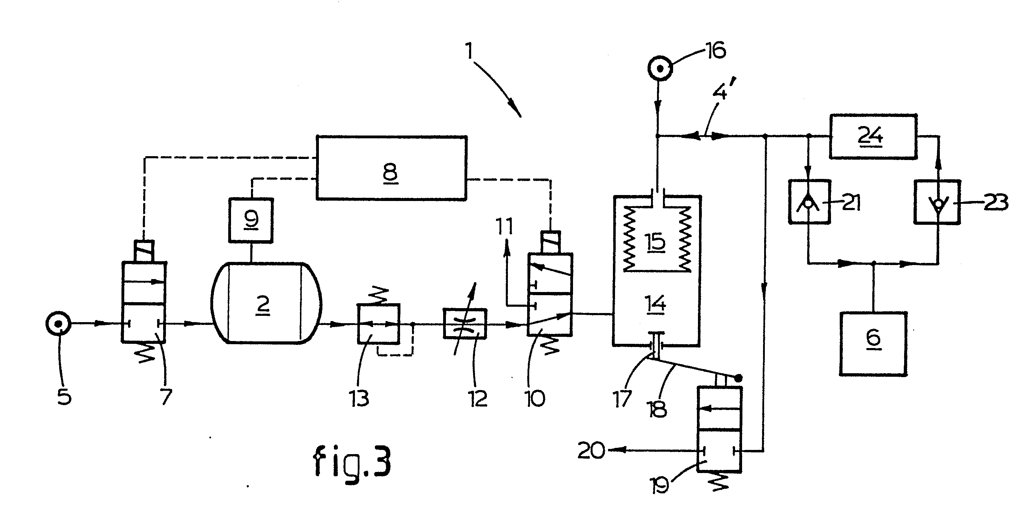

Fig. 3 shows a device 1, which substantially corresponds to the device 1 of fig. 2, wherein, however, the lung ventilator operates as 'semi-enclosed system'. This means that the air expired by the patient 6 is returned again through the line 4' to the bag 15 via a one way valve 23 and a carbon dioxide absorbing apparatus 24, while said expired air is replenished with new medical gas from the source 16.

In the devices operating according to the 'bag-in-bottle' principle the volume of the propellant delivered to the pressure chamber 14 at each breathing stroke will be larger than the quantity of medical gas supplied from the bag 15 to the patient 6. This is a result of the compliance of the pressure chamber 14. Therefore said compliance will have to be compensated by supplying more propellant than the quantity of the medical gas to be delivered. This compensation, however, can be controlled electronically in a simply yet accurate manner.

The invention is not restricted to the embodiments shown in the drawing by way of example, which can be varied in different ways within the scope of the invention.

C L A I M S

1. Device for measuring and delivering specified quantaties of compressible fluid, comprising a fluid container and adjusting means determining the quantity of fluid to be delivered, c h a r a c t e r i z e d in that the fluid container comprises at least one pressure vessel having a fixed volume, the pressure of the fluid therein being measurable by a pressure transducer, the adjustment means being valve members accommodated in a gas line before and after the pressure vessel, wherein during filling of the pressure vessel with fluid the valve member located before the pressure vessel is closed at a predetermined upper pressure and during emptying of the pressure vessel the valve member located after the pressure vessel is closed at a predetermined lower pressure by means of the pressure transducer.

2. Device according to claim 1, c h a r a c t e r i z e d in that a number of pressure vessels having different volumes are arranged in a parallel connection, the pressure vessels each being optionally applicable.

3. Device according to claim 1 or 2, c h a r a c t e r i z e d in that the valve members are electromagnetic actuated valves.

4. Device according to one of the preceding claims, c h a r a c t e r i z e d in that an adjustable throttle member is accommodated in the gas line after the pressure vessel.

5. Device according to claim 4, c h a r a c t e r i z e d in that the adjustable throttle member is a controllable restriction.

6. Device according to claim 4 or 5, c h a r a c t e r i z e d in that a pressure regulator is accommodated in the gas line between the pressure vessel and the adjustable throttle member.

7. Device according to one of the preceding claims, c h a r a c t e r i z e d in that the pressure vessel

0256171

contains a material having good heat conducting properties and having a large contacting area.

8. Device according to claim 7, c h a r a c t e r i z e d in that the heat conducting material is a porous mass.

9. Device according to claim 7 or 8, c h a r a c t e r i z e d in that the heat conducting material is copper wool.

10. Device according to one of the preceding claims, c h a r a c t e r i z e d in that the pressure transducer and the valve members are connected to a microcomputer.

11. Device according to one of the preceding claims, c h a r a c t e r i z e d in that the gas line after the pressure vessel is connected to a pressure chamber, containing a collapsible compressible bag which is closed with respect to the pressure chamber, the bag being adapted to be filled with a separate fluid which is able to be delivered by supplying fluid from the pressure vessel to the pressure chamber.

fig.1

fig.2

0256171

fig.3

European Patent Office

## DOCUMENTS CONSIDERED TO BE RELEVANT

| Category | Citation of document with indication, where appropriate, of relevant passages | Relevant to claim | CLASSIFICATION OF THE APPLICATION (Int Cl.4) |
|---|---|---|---|
| X | CA-A-1 109 766 (J.P. STRUPAT) * Page 2, lines 1,2; page 3, line 13 - page 4, line 12; page 4, line 17 - page 5, line 15; page 5, line 27 - page 8, line 20; figures 1-5 * | 1,6 | F 17 C 13/02 // A 61 M 16/00 |
| Y | | 3,4,10 | |
| Y | FR-A-2 352 557 (INSTITUT NATIONAL DE LA SANTE) * Page 1, lines 15-19; page 2, lines 14-32; figures 1,2 * | 3,4,10 | |
| A | FR-A-2 093 708 (LINDE) * Page 1, lines 1-14,24-33; page 2, line 29 - page 3, line 6; figures 1,2 * | 7 | |
| A | DE-A-2 055 744 (LKB MEDICAL) * Page 1, last paragraph - page 3, line 2; page 7, last paragraph - page 11, paragraph 2; figure 1 * | 11 | TECHNICAL FIELDS SEARCHED (Int. Cl.4) A 61 M F 17 C |
| L | NL-A-8 501 824 (HOEK'S MACHINE-EN ZUURSTOFFABRIEK) (Application date: 25-06-1985) | 1-11 | |

The present search report has been drawn up for all claims

| Place of search | Date of completion of the search | Examiner |
|---|---|---|
| THE HAGUE | 13-11-1987 | SIEM T.D. |